(19) Europäisches Patentamt / European Patent Office / Office européen des brevets

(11) **EP 3 324 827 B1**

(12) **EUROPÄISCHE PATENTSCHRIFT**

(45) Veröffentlichungstag und Bekanntmachung des Hinweises auf die Patenterteilung:
**20.10.2021 Patentblatt 2021/42**

(21) Anmeldenummer: **16739119.2**

(22) Anmeldetag: **14.07.2016**

(51) Int Cl.:
*A61B 5/00* (2006.01)   *A61B 5/0245* (2006.01)
*A61B 5/024* (2006.01)   *A61B 5/302* (2021.01)

(86) Internationale Anmeldenummer:
**PCT/EP2016/066784**

(87) Internationale Veröffentlichungsnummer:
**WO 2017/012987 (26.01.2017 Gazette 2017/04)**

(54) **SYSTEM, VERFAHREN UND COMPUTERPROGRAMM ZUR KAPAZITIVEN ERFASSUNG VON ELEKTRISCHEN BIOSIGNALEN**

SYSTEM, METHOD AND COMPUTER PROGRAM FOR CAPACITIVELY RECORDING ELECTRICAL BIO-SIGNALS

SYSTÈME, PROCÉDÉ ET LOGICIEL POUR LA DÉTECTION CAPACITIVE DES SIGNAUX BIOLOGIQUES ÉLECTRIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.07.2015 DE 102015111658**

(43) Veröffentlichungstag der Anmeldung:
**30.05.2018 Patentblatt 2018/22**

(73) Patentinhaber: **Capical GmbH**
**38106 Braunschweig (DE)**

(72) Erfinder:
- **OEHLER, Martin**
  **38108 Braunschweig (DE)**
- **ELENSKIY, Ilya**
  **38106 Braunschweig (DE)**

(74) Vertreter: **Gramm, Lins & Partner**
**Patent- und Rechtsanwälte PartGmbB**
**Freundallee 13a**
**30173 Hannover (DE)**

(56) Entgegenhaltungen:
**EP-A1- 2 101 408      WO-A1-2015/045763**
**WO-A2-2006/065765**

- SERTEYN A ET AL: "Motion Artifacts in Capacitive ECG Measurements: Reducing the Combined Effect of DC Voltages and Capacitance Changes Using an Injection Signal", IEEE TRANSACTIONS ON BIOMEDICAL ENGINEERING, IEEE SERVICE CENTER, PISCATAWAY, NJ, USA, Bd. 62, Nr. 1, 15. August 2014 (2014-08-15), Seiten 264-273, XP011568272, ISSN: 0018-9294, DOI: 10.1109/TBME.2014.2348178 [gefunden am 2014-12-18]
- SERTEYN A ET AL: "ECG reconstruction based on the injection of a multi-frequency signal in capacitive measurement systems", 2014 36TH ANNUAL INTERNATIONAL CONFERENCE OF THE IEEE ENGINEERING IN MEDICINE AND BIOLOGY SOCIETY, IEEE, 26. August 2014 (2014-08-26), Seiten 1864-1867, XP032674931, DOI: 10.1109/EMBC.2014.6943973 [gefunden am 2014-11-02]
- STEPHAN HEUER ET AL: "Unobtrusive in-vehicle biosignal instrumentation for advanced driver assistance and active safety", BIOMEDICAL ENGINEERING AND SCIENCES (IECBES), 2010 IEEE EMBS CONFERENCE ON, IEEE, 30. November 2010 (2010-11-30), Seiten 252-256, XP031936758, DOI: 10.1109/IECBES.2010.5742238 ISBN: 978-1-4244-7599-5

**Beschreibung**

Die Erfindung betrifft ein System zur kapazitiven Erfassung von elektrischen Biosignalen von einer Biosignalquelle, wobei das System wenigstens eine kapazitive Messelektrode und wenigstens eine mit der Messelektrode gekoppelte elektronische Auswerteeinheit zur Auswertung der elektrischen Signale der Messelektrode aufweist, und wobei das System ferner Mittel zur Überwachung der Güte der kapazitiven Kopplung der Messelektrode mit der Biosignalquelle aufweist. Die Erfindung betrifft ferner ein Verfahren zur Bestimmung der Güte der Kopplung wenigstens einer Messelektrode eines solchen Systems mit einer Biosignalquelle sowie ein Computerprogramm mit Programmcodemitteln, eingerichtet zur Ausführung des Verfahrens.

Allgemein betrifft die Erfindung das Gebiet der Erfassung von elektrischen Biosignalen durch kapazitive Erfassung, d.h. ohne Herstellung eines galvanischen Kontakts mit der Haut. Die Biosignalquelle kann dabei jedes Lebewesen sein, insbesondere ein Mensch. Als Biosignale können z.B. EKG-Signale, EEG-Signale und ähnliche elektrisch erfassbare Signale aufgenommen werden. Die Biosignale können z. B. für medizinische Diagnosezwecke ausgewertet werden, oder auch als sonstige Indikatorsignale, die z.B. in Fahrzeugsystemen weiter ausgewertet werden.

Es gibt bereits Vorschläge, Fahrzeugsitze von Automobilen mit textilen kapazitiven Elektroden auszurüsten, um auf diese Weise die Fahrtauglichkeit eines Fahrers jederzeit zu überwachen. Aus der DE 10 2013 108 810 A1 geht ein Vorschlag für eine solche textile kapazitive Elektrode hervor.

Bei solchen kapazitiven Erfassungen von Biosignalen ist es für die Evaluierung der erfassten Signale von Bedeutung zu wissen, wie gut die Signalübertragung von der Biosignalquelle auf die Messelektrode ist. Zum Beispiel kann es bei in den Fahrzeugsitz eingebauten Messelektroden passieren, dass die zu erfassende Person ungünstig auf dem Sitz platziert ist oder sonstige äußere Faktoren für eine schlechte Signalübertragung sorgen. Ein solcher Fall muss davon unterscheidbar sein, dass die Person, d.h. die Biosignalquelle, aus gesundheitlichen Gründen keine oder nur geringe Biosignale an die Messelektrode abgibt.

Aus der EP 2 101 408 A1 sind Front-End Verstärker und Einzeldrahtmessgeräte bekannt.

Es gibt daher bereits Vorschläge, die Güte der kapazitiven Kopplung der Messelektrode mit der Biosignalquelle zu überwachen. Aus der WO 2015/075692 A1 geht der Vorschlag hervor, über bestimmte Messelektroden sogenannte Trägersignale (carrier signals) in die Biosignalquelle einzuspeisen und über andere Messelektroden zusammen mit den eigentlichen zu erfassenden Signalen auszuwerten. Dieser Vorschlag erscheint für eine praktische Umsetzung in Systemen mit vielen Messelektroden relativ aufwendig, da eine entsprechend große Anzahl von Trägersignalen generiert werden muss. Zudem kann es zu einem Übersprechen zwischen verschiedenen Messelektroden kommen, wenn mehrere Messelektroden gleichzeitig ein Trägersignal injizieren.

Der Erfindung liegt daher die Aufgabe zugrunde, ein System zur kapazitiven Erfassung von elektrischen Biosignalen anzugeben, bei dem die Güte der kapazitiven Kopplung der Messelektrode mit der Biosignalquelle weniger aufwendig bestimmt werden kann. Ferner soll ein vorteilhaftes Verfahren zur Bestimmung der Güte der Kopplung wenigstens einer Messelektrode eines solchen Systems mit einer Biosignalquelle angegeben werden, außerdem ein Computerprogramm mit Programmcodemitteln zur Ausführung des Verfahrens.

Diese Aufgabe wird gemäß Anspruch 1 gelöst durch ein System zur kapazitiven Erfassung von elektrischen Biosignalen von einer Biosignalquelle, wobei das System wenigstens eine kapazitive Messelektrode und wenigstens eine mit der Messelektrode gekoppelte elektronische Auswerteeinheit zur Auswertung der elektrischen Signale der Messelektrode aufweist, und wobei das System ferner Mittel zur Überwachung der Güte der kapazitiven Kopplung der Messelektrode mit der Biosignalquelle aufweist, wobei die Mittel zur Überwachung der Güte der kapazitiven Kopplung zusätzlich zu der Messelektrode oder den Messelektroden wenigstens zwei elektrisch voneinander separierte Injektionselektroden zur Einspeisung von Injektionssignalen in die wenigstens eine Messelektrode über die Biosignalquelle aufweisen, wobei das System Signalgeneratoren zur Erzeugung eines ersten Injektionssignals und eines vom ersten Injektionssignal unterschiedlichen zweiten Injektionssignals aufweist, die mittels der Injektionselektroden über die Biosignalquelle in die Messelektrode eingespeist werden, und wobei das System eine Bestimmungseinheit aufweist, die dazu eingerichtet ist, anhand der über die Messelektrode empfangenen Signale anhand der darin enthaltenen, von den ersten und zweiten Injektionssignalen herrührenden Signalanteilen die Güte der kapazitiven Kopplung der Messelektrode mit der Biosignalquelle zu bestimmen. Durch das Einführen von zusätzlichen Injektionselektroden, die zusätzlich zu der Messeelektrode oder den Messelektroden des Systems vorhanden sind und dementsprechend nicht als Messelektroden dienen, d.h. nicht zur Erfassung von elektrischen Biosignalen, können festgelegte Einspeisepfade für Injektionssignale bereitgestellt werden, die entsprechend ihrer festgelegten Funktion nur mit der dafür erforderlichen elektronischen Beschaltung versehen werden müssen. Die Injektionselektroden können insbesondere als Masseelektroden (kapazitiv oder galvanisch an den Körper gekoppelt) im Sinne von DRL-Elektroden, wie sie von EKG-Systemen her bekannt sind, ausgebildet sein (DRL - driven right leg). Auf diese Weise können die Injektionselektroden als Elektroden ausgebildet sein, die im Unterschied zu den Messelektroden bereits aktiv durch jeweilige Verstärker getrieben sind. Dementsprechend ist der zusätzliche Aufwand für die Einspeisung der Injektionssignale relativ gering. So kann z.B. ein Injektionssignal in Form eines Sinussignals dem bei einer DRL-Elektrode ohnehin zugeführten Gleichtaktunterdrückungssignal mittels eines Addierers mit wenig Aufwand hinzugefügt werden, zumindest mit deutlich geringerem Aufwand, als bei einer Modifikation von Messelektroden nötig wäre.

Das erfindungsgemäße System hat den Vorteil, dass über die zwei voneinander separierten Injektionselektroden zwei unterschiedliche Injektionssignale eingespeist werden können, die über die Biosignalquelle wiederum mittels der Messelektrode bzw. der Messelektroden erfassbar sind und voneinander unterscheidbar sind. Dementsprechend kann anhand der über die Messelektrode empfangenen Signale anhand der darin enthaltenen, von den ersten und zweien Injektionssignalen herrührenden Signalanteilen die Güte der kapazitiven Kopplung der Messelektrode mit der Biosignalquelle bestimmt werden. Vorteilhafterweise sind unabhängig von der Zahl der verwendeten Messelektroden des Systems immer nur die wenigstens zwei Injektionselektroden mit den voneinander unterschiedlichen Injektionssignalen erforderlich. Es lassen sich damit für jede Messelektrode deren Güte der kapazitiven Kopplung mit der Biosignalquelle anhand der erfassten Signale bestimmen. Hierzu kann die Bestimmungseinheit z.B. ein Filter zum Herausfiltern der von den ersten und den zweiten Injektionssignalen herrührenden Signalanteile aufweisen.

Als Ergebnis der Bestimmung der Güte der kapazitiven Kopplung der Messelektrode mit der Biosignalquelle kann z.B. ein Zahlenwert bestimmt werden, z.B. ein Zahlenwert, der die Kopplungskapazität wiedergibt, oder nach entsprechender Vorauswertung auch eine Gut/Schlecht-Information, die angibt, ob die über eine Messelektrode erfassten elektrischen Biosignale der Biosignalquelle sinnvoll auswertbar sind oder nicht. Insbesondere kann auch der zeitliche Verlauf der auf diese Weise bestimmten Güte der kapazitiven Kopplung ausgewertet werden.

Gemäß einer vorteilhaften Weiterbildung der Erfindung sind eine, mehrere oder alle Messelektroden des Systems als textile kapazitive Elektroden ausgebildet, die in einer Textilstruktur eingebettet sind. Die Textilstruktur kann z.B. Teil eines Sitz-Möbelstücks oder ein Sitz in einem Verkehrsmittel sein, z.B. in einem Kraftfahrzeug, einem Flugzeug oder ähnlichem. Die Messelektroden können dabei insbesondere in eine Rückenlehne des Sitzes eingebettet sein. Des Weiteren können die Elektroden in eine Liege oder ein Bett integriert werden.

Gemäß einer vorteilhaften Weiterbildung der Erfindung sind die wenigstens zwei Injektionselektroden als textile kapazitive Elektroden ausgebildet, die in einer Textilstruktur eingebettet sind. Die Injektionselektroden können z.B. in einer Sitzfläche eines Sitzes eingebettet sein. Hierdurch kann von einer jederzeit relativ guten Kopplung zur Biosignalquelle ausgegangen werden.

Auf diese Weise kann eine mit den Messelektrode und/oder den Injektionselektroden bestückte Textilstruktur angegeben werden, die auf einfache und kostengünstige Weise bereitgestellt werden kann. Durch die Ausführung der Elektroden als textile kapazitive Elektroden tragen diese wenig auf und stören den Sitzkomfort nicht.

Gemäß einer vorteilhaften Weiterbildung der Erfindung ist vorgesehen, dass eine, mehrere oder alle Messelektroden des Systems als flexible kapazitive Elektroden mit leitfähigen Strukturen ausgebildet sind, wobei die leitfähigen Strukturen als gedruckte Strukturen ausgebildet sind. Dementsprechend können die leitfähigen Strukturen in einem Druckverfahren realisiert werden. Die leitfähigen Strukturen können z.B. als Siebdruck-gedruckte Strukturen ausgebildet sein, d.h. mittels Siebdruck realisiert werden. Es sind auch andere Druckverfahren vorteilhaft anwendbar, wie z.B. Offsetdruck, Tiefdruck oder Flexodruck. Hierdurch können besonders flexibel anpassungsfähige kapazitive Elektroden bereitgestellt werden, die zudem kostengünstig in großen Stückzahlen herstellbar sind.

Gemäß einer vorteilhaften Weiterbildung der Erfindung ist in die eine Injektionselektrode nur das erste Injektionssignal eingespeist und in die andere Injektionselektrode eine Überlagerung aus dem ersten und dem zweiten Injektionssignal eingespeist. Gemäß einer vorteilhaften Weiterbildung der Erfindung ist in beide Injektionselektroden zusätzlich ein Gleichtaktunterdrückungssignal eingespeist.

Gemäß einer vorteilhaften Weiterbildung der Erfindung ist die Bestimmungseinheit zur Bestimmung der Güte der kapazitiven Kopplung anhand der Amplitudenwerte und der Phasenlagen der über die Messelektrode empfangenen Signalanteile des ersten und des zweiten Injektionssignals eingerichtet.

Gemäß einer vorteilhaften Weiterbildung der Erfindung ist das System zur Bestimmung der Herzfrequenz oder einer daraus abgeleiteten Größe der Biosignalquelle eingerichtet.

Gemäß einer vorteilhaften Weiterbildung der Erfindung sind der Bestimmungseinheit als weitere Eingangsgrößen Messwerte der über die erste und die zweite Injektionselektrode mittels der zugeführten Injektionssignale eingespeisten Ströme zugeführt und die Bestimmungseinheit ist zur Bestimmung der Güte der kapazitiven Kopplung unter Berücksichtigung der zugeführten Messwerte der in die erste und die zweite Injektionselektrode mittels der zugeführten Injektionssignale eingespeisten Ströme eingerichtet. Die eingespeisten Ströme können z.B. durch Messwiderstände (Shunt) ermittelt werden. Auf diese Weise können weitere elektrische Messgrößen für die Auswertung der mittels der Injektionselektroden eingespeisten Größen erfasst werden, so dass die rechnerische Bestimmung der Güte der kapazitiven Kopplung der Messelektrode weiter vereinfacht wird.

Die eingangs genannte Aufgabe wird ferner gelöst durch ein Verfahren zur Bestimmung der Güte der Kopplung wenigstens einer Messelektrode eines Systems der zuvor beschriebenen Art mit einer Biosignalquelle, mit den Schritten:

a) Zeitlich überlappendes oder gleichzeitiges Einspeisen eines ersten Injektionssignals und eines davon verschiedenen zweiten Injektionssignals über die Biosignalquelle in die wenigstens eine Messelektrode.

b) Auswerten der von der wenigstens eine Messelektrode empfangenen elektrischen Signale,

c) Bestimmen der Güte der kapazitiven Kopplung anhand der in den empfangenen Signalen enthaltenen Signalanteile des ersten und des zweiten Injektionssignals.

Auch hierdurch können die zuvor erläuterten Vorteile realisiert werden.

Die eingangs genannte Aufgabe wird außerdem gelöst durch ein Computerprogramm mit Programmcodemitteln, eingerichtet zur Ausführung des Verfahrens der zuvor erläuterten Art, wenn das Computerprogramm auf einem Rechner des Systems ausgeführt wird. Der Rechner des Systems kann z.B. ein Rechner der Bestimmungseinheit sein. Auch hierdurch werden die zuvor erläuterten Vorteile realisiert.

Die Erfindung wird nachfolgend anhand von Ausführungsbeispielen unter Verwendung von Zeichnungen näher erläutert. Der Schutzumfang der Erfindung ist durch die beigefügten Ansprüche definiert.

Es zeigen

Figur 1 ein erfindungsgemäßes System in schematischer Darstellung und
Figur 2 den mehrlagigen Aufbau einer Textilelektrode und
Figur 3 eine Signalerfassungsschaltung einer Messelektrode und
Figur 4 ein Ersatzschaltbild eines 2-Kanal-Systems und
Figur 5 ein Ersatzschaltbild der Injektionselektroden.

In den Figuren werden gleiche Bezugszeichen für einander entsprechende Elemente verwendet.

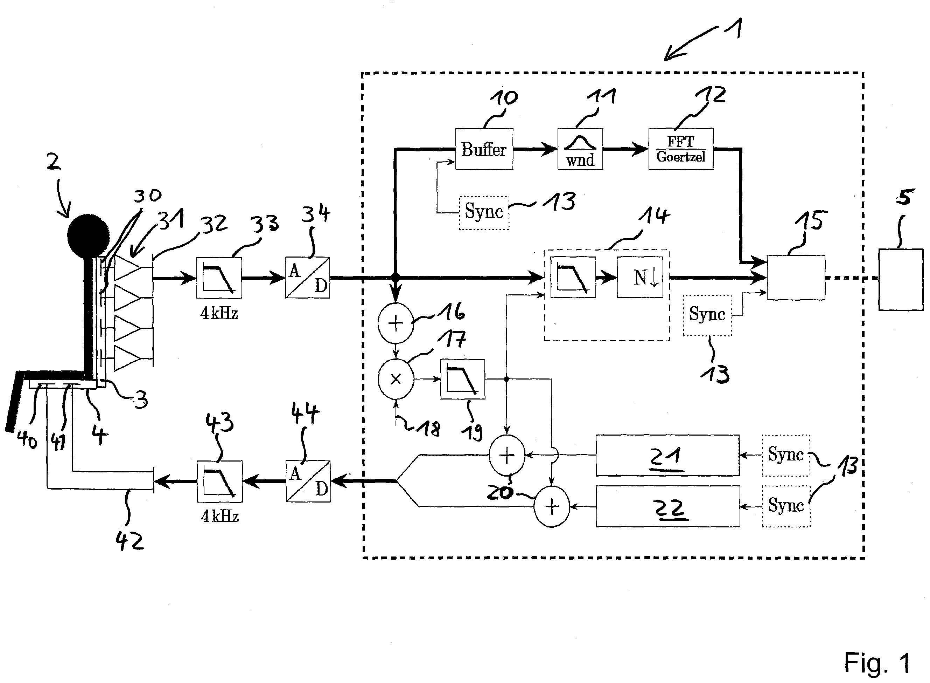

Das in Figur 1 dargestellte System dient zur kapazitiven Erfassung von elektrischen Biosignalen von einer Biosignalquelle 2, z.B. einem Menschen. Hierfür ist ein textilbezogener Sitz, z.B. ein Fahrzeugsitz, mit entsprechenden kapazitiven Messelektroden und Injektionselektroden bestückt. Beispielhaft ist dargestellt, dass in eine Rückenlehne 3 des Sitzes vier Messelektroden 30 in Form von textilen kapazitiven Elektroden integriert sind. In eine Sitzfläche 4 des Sitzes sind zwei Injektionselektroden 40, 41 integriert, die ebenfalls als textile kapazitive Elektroden ausgebildet sein können. Die Injektionselektroden 40, 41 sind über separate elektrische Leitungen mit Einrichtungen 43, 44 verbunden, die in der Figur 1 nur einfach dargestellt sind, aber separat für jede Injektionselektrode realisiert sind. Bei der Einrichtung 43 handelt es sich um einen Tiefpass, z.B. mit einer Grenzfrequenz von 4 kHz. Die Einrichtung 44 ist als Digital/Analog-Wandler ausgebildet, der ein von einer Bestimmungseinheit 1 zugeführtes Digitalsignal in einen analogen Spannungswert wandelt und über das Tiefpassfilter 43 an die jeweilige Injektionselektrode 40, 41 ausgibt.

Die Messelektroden 30 sind über jeweilige Signalverstärker 31, die auch in die jeweilige textile Elektrode integriert sein können, mit weiteren Signalverarbeitungsmitteln 33, 34 verbunden. Die Messelektroden 30 bzw. ihre Signalverstärker 31 können jeweils über einen einzelnen, separaten Signalpfad über Signalverarbeitungsmittel 33, 34 mit der Bestimmungseinheit 1 verbunden sein, oder, wenn der Schaltungsaufwand verringert werden soll, über einen Multiplexer 32 jeweils an dieselben Signalverarbeitungsmittel 33, 34 geschaltet werden. Das Signalverarbeitungsmittel 33 kann als Tiefpassfilter ausgebildet sein, z.B. mit einer Grenzfrequenz von 4 kHz. Das Signalverarbeitungsmittel 34 kann als Analog/Digital-Wandler ausgebildet sein.

Durch die jeweilige Analog/Digital-Wandlung bzw. Digital/Analog-Wandlung kann in der Bestimmungseinheit 1 die Signalverarbeitung vollständig digital erfolgen, mit dem Vorteil, dass relativ günstig komplexe Signalverarbeitungsalgorithmen realisiert werden können.

Die mit dem Analog/Digital-Wandler 34 bzw. den Digital/Analog-Wandlern 44 verbundene Bestimmungseinheit 1 weist folgenden Aufbau auf. Die über den Analog/Digital-Wandler 34 erfassten, digitalisierten Signale der Messelektrode 30 werden drei unterschiedlichen Auswertungspfaden in der Bestimmungseinheit 1 zugeführt, und zwar einem Pfad für die Auswertung der von den Injektionssignalen herrührenden Signalanteile, einem Pfad für die Ermittlung der eigentlichen Nutzsignale, nämlich der Biosignale der Biosignalquelle, sowie einem Pfad, der zur Gleichtaktunterdrückung dient. Zunächst sei auf den Pfad zur Auswertung der von den Injektionssignalen herrührenden Signalanteile eingegangen. Hierfür ist zunächst ein Puffer 10 vorhanden, indem die eingehenden Daten zunächst einmal blockweise gepuffert werden, z.B. mit einer Blockgröße von 728 Messwerten. Die Blockgröße wird dabei insbesondere so gewählt, dass in einem Block jeweils volle Perioden des ersten und des zweiten Injektionssignals gespeichert sind.

In einem Block 11 werden die Signalanteile mit einer nicht-rechteckigen Fensterfunktion vorverarbeitet, z.B. einem Hanning-Fenster. In einem darauffolgenden Digitalfilter 12 erfolgt eine weitere Filterung, z.B. mittels einer schnellen Fourier-Transformation (FFT) oder einem Goertzel-Algorithmus. Der Goertzel-Algorithmus erlaubt die effiziente Bestimmung von ausgewählten Frequenzanteilen. Mit den auf diese Weise bestimmten Daten kann in einem Block 15 die Güte der kapazitiven Kopplung der Messelektrode mit der Biosignalquelle bestimmt werden, z.B. in Form der Kopplungskapazität. Die Ergebnisse der Gütebestimmung können z.B. auf ein Anzeigegerät, z.B. einem Bildschirm 5, ausgegeben werden oder einer weiteren Verarbeitung zugeführt werden.

Über den in Figur 1 in der Bestimmungseinheit 1 etwa in der Mitte dargestellten Filterblock 14 werden die EKG-Signale aus den zugeführten Signalen der Messelektrode herausgefiltert. Dies kann z.B. durch ein Tiefpass-Filter erfolgen.

Für die Gleichtaktunterdrückung ist vorgesehen, das zugeführte, digitalisierte Messsignal über einen Summierer 16 zunächst aufzusummieren. Hierdurch wird das Gleichtaktsignal gewonnen. In einem Multiplizierer 17 kann das zuvor bestimmte Gleichtaktsignal noch mit einem Verstärkungsfaktor 18 verstärkt werden, z.B. im Bereich von 0 bis 40 dB. Das hierdurch gebildete Signal wird anschließend einem weiteren Filter 19 zugeführt. Das aus dem Filter 19 erzeugte Signal wird einerseits dem Filterblock 14 zugeführt, außerdem zwei Summierern 20.

In dem unten in der Bestimmungseinheit 1 dargestellten Blöcken wird in zwei Signalgeneratoren 21, 22 das erste und das zweite Injektionssignal erzeugt. Das erste Injektionssignal kann z.B. eine Frequenz von 1120 Hz bei 100 mV Amplitude aufweisen, das zweite Injektionssignal eine Frequenz von 1040 Hz bei 12,5 mV Amplitude. So kann der erste Signalgenerator 21 dazu ausgebildet sein, direkt eine Überlagerung aus dem ersten und dem zweiten Injektionssignal abzugeben, während der andere Signalgenerator 22 nur das erste Injektionssignal abgibt. In den Summierern 20 wird den jeweiligen Injektionssignalen das durch das Filter 19 abgegebene Signal zur Gleichtaktunterdrückung zugemischt. Die entsprechenden, bis dahin digital vorliegenden Signale werden über die bereits erwähnten Digital/Analog-Wandler 44 in Analogsignale gewandelt und über die Filter 43 in die Injektionselektroden 40, 41 separat voneinander eingespeist.

Für die Dimensionierung der Injektionssignale wurde ein Kompromiss gefunden, der es erlaubt, die Injektionssignale auf möglichst nahe beieinanderliegenden Frequenzen zu platzieren, eine gute Demodulationsrate zu bieten und dabei eine für geeignete Präzisions-Analog-Digital-Wandler und verfügbarere Mikrocontroller erreichbare Abtastraten erlaubt. Außerdem müssen die Injektionsfrequenzen hoch genug liegen, um mit einem einfachen Tiefpassfilter ausreichend gegenüber dem Nutzsignal (dem EKG-Signal) unterdrückt werden zu können. Hierdurch ist zudem auch eine Abgrenzung von Bewegungsartefakten möglich, die im Bereich unterhalb 20 Hz liegen.

Auch die Amplitude der Injektionssignale stellt einen Kompromiss zwischen gutem Signal-Rausch-Verhältnis und möglichst niedriger Ordnung der Tiefpassfilter dar, um eine einfache Signalverarbeitung zu ermöglichen.

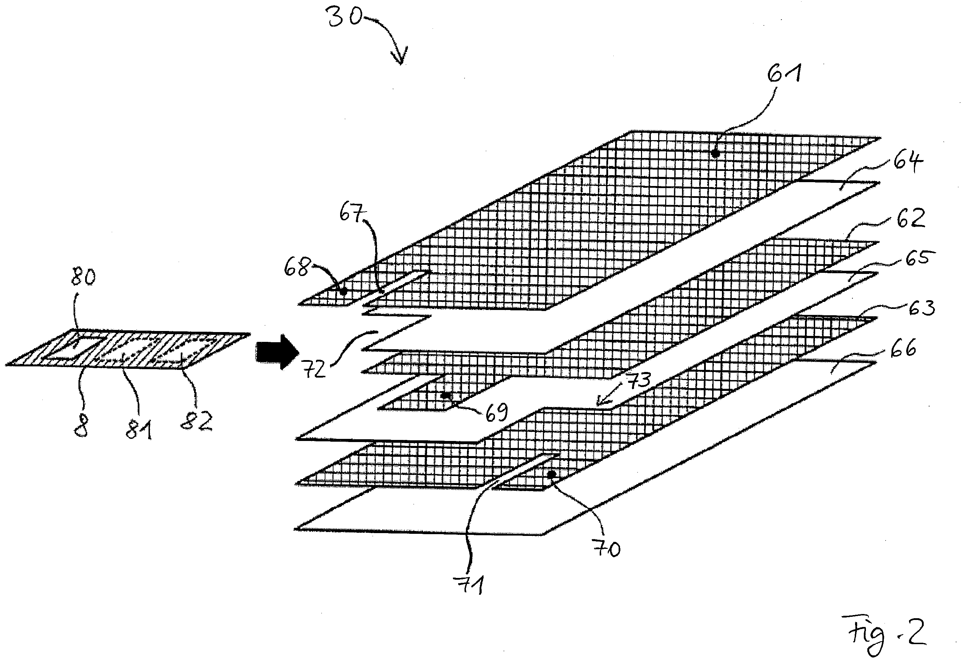

Die Figur 2 zeigt beispielhaft eine Textilelektrode, wie sie als Messelektrode 30 oder auch Injektionselektrode 40, 41 genutzt werden kann. Es sei an dieser Stelle noch einmal darauf hingewiesen, dass die Erfindung nicht auf textile Elektroden beschränkt ist, sondern mit jeder Art kapazitiver Elektrode nutzbar ist.

Die Figur 2 zeigt die Elektrode 1 mit den einzelnen Schichten in einer isometrischen Ansicht, bevor die Schichten zusammengeklebt sind. Erkennbar sind drei elektrisch leitfähige Schichten 61, 62, 63 aus einem elektrisch leitfähigen Textilmaterial sowie drei Isolationsschichten 64, 65, 66 aus einem isolierenden Textilmaterial. Die oberste elektrisch leitfähige Schicht 61 ist die Sensorschicht der Elektrode, die zur kapazitiven Einkopplung des mittels der Elektrode zu messenden Signals dient. Die mittlere elektrisch leitfähige Schicht 62 ist eine Guardschicht, die zur Abschirmung der Sensorschicht 61 gegen äußere Störeinflüsse, insbesondere ESD-Einflüsse, dient. Die untere elektrisch leitfähige Schicht 63 ist eine Bezugspotentialschicht, die mit einem Bezugspotential zu verbinden ist. Die Sensorschicht 61 weist an einer Ecke eine Aussparung 67 auf, durch die eine Kontaktlasche 68 zur elektrischen Kontaktierung der Sensorschicht 61 gebildet wird. Die Guardschicht 62 weist eine Kontaktlasche 69 auf, die dadurch gebildet ist, dass links und rechts von der Kontaktlasche 69 Stücke vom Textilmaterial der Guardschicht 62 abgetrennt worden sind. Die Kontaktlasche 69 dient zur elektrischen Kontaktierung der Guardschicht 62. Die Bezugspotentialschicht 63 ist vergleichbar ausgebildet wie die Sensorschicht 61, jedoch mit einer Kontaktlasche 70 auf der gegenüberliegenden Seite. Die Kontaktlasche 70 wird in Folge einer Aussparung 71, die aus dem Textilmaterial der Bezugspotentialschicht 63 herausgeschnitten ist, gebildet. Die oberste Isolationsschicht 64 weist an einer Ecke eine Aussparung 72 auf, die unterhalb der Kontaktlasche 68 liegt. Die mittlere Isolationsschicht 65 weist an einer gegenüberliegenden Ecke derselben Seite eine Aussparung 73 auf. Die Aussparung 73 überlappt mit der Kontaktlasche 70. Die unterste Isolationsschicht 66 weist keine solchen Aussparungen auf. Die Schichten 61 - 66 können z. B. durch Laserschneiden in die beschriebene und dargestellte äußere Kontur gebracht werden.

Die äußere Form der Elektrode 1 bzw. der einzelnen Schichten 61 - 66 muss nicht unbedingt, wie in Fig. 2 dargestellt, im Wesentlichen rechteckig sein, sondern kann jede andere gewünschte Form einnehmen, wie z. B. oval, rechteckig mit abgerundeten Ecken oder kreisrund.

In dem in Fig. 2 dargestellten mehrschichtigen Aufbau wird in dem Bereich, in dem die Kontaktlaschen 68, 69, 70 vorhanden sind, eine Verstärkerelektronik 83, die zur Verstärkung der von der kapazitiven Elektrode 1 abgegebenen elektrischen Signale dient, integriert. Die Verstärkerelektronik 83 wird dabei zwischen der oberen Isolationsschicht 64 und der Guardschicht 62 angeordnet. Dies ist jedoch nur ein Beispiel für eine mögliche Anordnung, auch andere Positionierungen der Verstärkerelektronik 83 sind vorteilhaft möglich. Die Verstärkerelektronik 83 weist an der in Fig. 2 oben dargestellten Seite eine elektrische Anschlussfläche 80 auf, und an der unteren Seite zwei weitere elektrische Anschlussflächen 81, 82, z. B. in Form von Kontaktpads. In Folge der Aussparungen 72, 73 kann die Kontaktlasche 68 elektrisch mit der Anschlussfläche 80 verbunden werden, die Kontaktlasche 69 mit der Anschlussfläche 81 und die Kontaktlasche 70 mit der Anschlussfläche 82. In Folge der in den übrigen Bereichen überlappenden Isolationsschichten 64, 65, 66 kann es nicht zu ungewollten Kurzschlüssen oder Fehlkontaktierungen kommen.

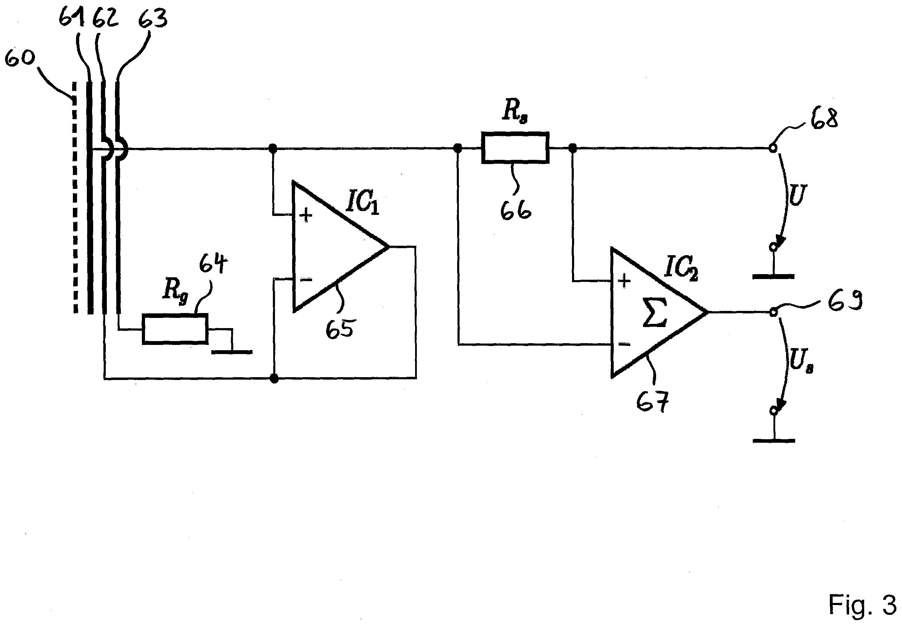

Die Figur 3 zeigt links beispielhaft eine Masseelektrode mit dem zuvor beschriebenen mehrschichtigen Aufbau, der hinter der textilen Oberfläche 60 der Rückenlehne 3 angeordnet ist. Die Bezugspotentialschicht 63 ist über einen Widerstand 64 mit der Systemmasse verbunden. Die Sensorschicht 61 ist über eine Leitung zunächst über einen Verstärker, z.B. ein Operationsverstärker 65, mit der Guardschicht 62 verbunden. Ferner wird für die Erfassung der bereits erläuterten, über die Messelektrode zu erfassenden Signale die Sensorschicht 61 mit einem Messanschluss 68 verbunden, an dem das mittels der Signalauswertemittel 33, 34 zu erfassende Messsignal anliegt. Um zusätzlich eine Strommessung bzgl. der Injektionselektroden 40, 41 durchführen zu können, ist in der Leitung von der Sensorschicht 61 zum Messanschluss 68 ein Messwiderstand in Form eines Shunt 66 vorhanden. Die über dem Shunt 66 abfallende Spannung, die ein Indikator für den dadurch fließenden Strom ist, wird über einen Verstärker 67 verstärkt und an einem Ausgangsanschluss 69 abgegeben. Das am Ausgangsanschluss 69 anstehende Signal wird, ggf. mit einer vorherigen Filterung, ebenfalls über Analog/Digital-Wandler der Bestimmungseinheit 1 zugeführt und dort weiter verarbeitet.

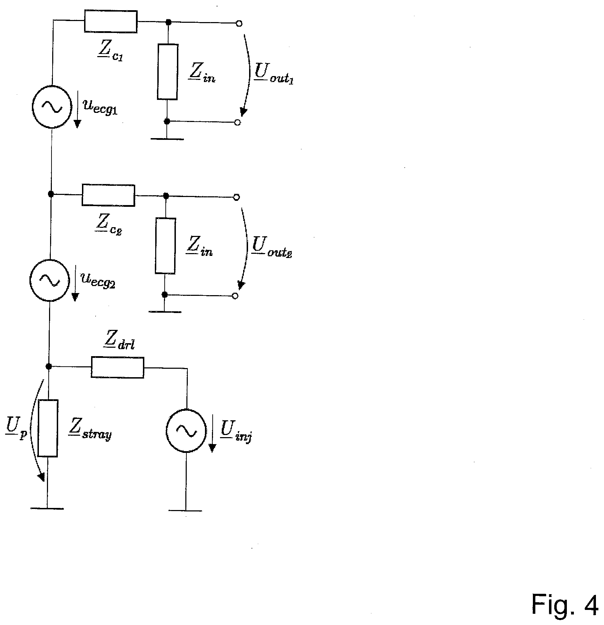

Die Bestimmung der Güte der kapazitiven Kopplung, z.B. in Form einer Koppelimpedanz, kann wie folgt erfolgen. Hierbei wird von dem in dem in Figur 4 dargestellten Ersatzschaltbild und dem dort angegebenen elektrischen Größen ausgegangen.

In Figur 4 ist das Ersatzschaltbild eines 2-Kanal-Systems (zwei Messelektroden) mit DRL-Signalinjektion dargestellt. Zusätzlich ist hier die parasitäre Impedanz $\underline{Z}_{stray}$ berücksichtigt. Sie entsteht durch die Streukapazitäten zwischen der Biosignalquelle und Objekten der Außenwelt sowie zwischen dem Messsystem und der Außenwelt. Diese ist besonders erheblich bei nicht isolierter Stromversorgung des Messsystems aus dem Netz. $\underline{U}_p$ stellt das an der Biosignalquelle anliegende Spannungssignal gegenüber der Systemmasse dar. Für die Koppelimpedanz einer Elektrode gilt:

$$\underline{U}_{out} = \underline{U}_p \frac{\underline{Z}_{in}}{\underline{Z}_{in} + \underline{Z}_c} \tag{3.11}$$

$$\Leftrightarrow \quad \underline{Z}_c = \underline{Z}_{in} \left( \frac{\underline{U}_p}{\underline{U}_{out}} - 1 \right) \tag{3.12}$$

Hieraus können abhängig von der Kreisfrequenz w des Injektionssignales die Kapazität und der Widerstand bestimmt werden:

$$C_c = \frac{\Im(\underline{Y}_c)}{\omega} = \frac{\Im(\frac{1}{\underline{Z}_c})}{\omega} \tag{3.13}$$

$$R_c = \frac{1}{\Re(\underline{Y}_c)} = \frac{1}{\Re(\frac{1}{\underline{Z}_c})} \tag{3.14}$$

$$\tag{3.15}$$

Das Modell zeigt jedoch, dass die Spannung $\underline{U}_p$ von den Impedanzen $\underline{Z}_{stray}$, $\underline{Z}_{drl}$ und $\underline{Z}_{ci}$ beeinflusst wird. Sie kann mit den vorhandenen Messdaten nicht eindeutig bestimmt werden.

Um $\underline{U}_p$ bestimmen zu können, wird mindestens eine weitere injizierende Elektrode benötigt.

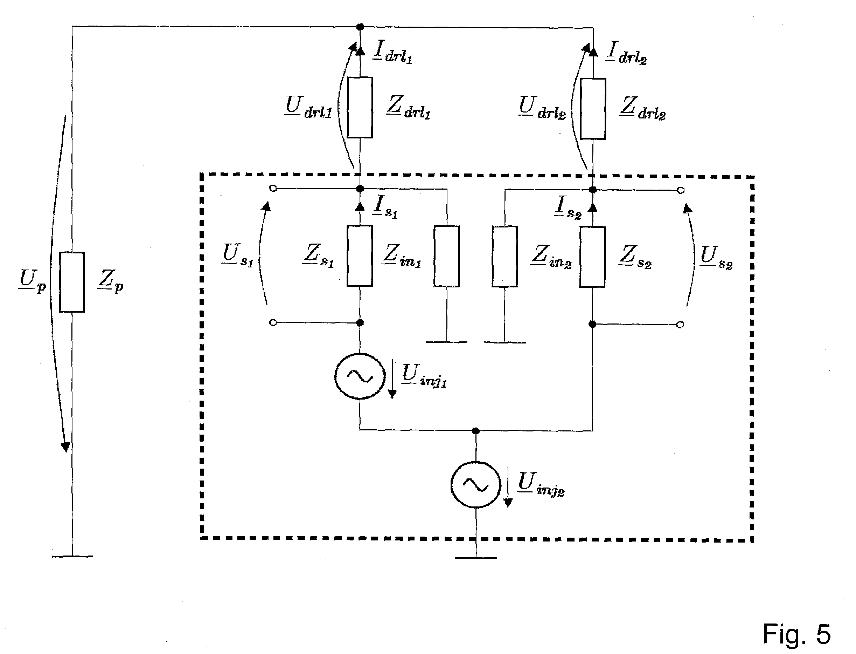

Dazu kann die DRL-Elektrode in zwei getrennte Flächen geteilt werden, um die Injektionselektroden zu bilden. Im Unterschied zu der Teilung der Messelektroden birgt es keinen Nachteil für die Signalqualität, da bei der DRL-Elektrode lediglich die Gesamtkapazität der beiden Flächen maximiert werden muss. Das entsprechende Ersatzschaltbild ist in Figur 5 zu sehen, die Elektroden- und Streukapazitäten sind dabei zu der Impedanz $\underline{Z}_p$ zusammengefasst. Es werden zwei Injektionssignale eingespeist: $\underline{U}_{inj2}$ mit der Kreisfrequenz $\omega 2$ über beide Flächen und $\underline{U}_{inj1}$ mit der Kreisfrequenz $\omega_1$ nur über die erste Fläche. Die beiden Teile der Elektrode enthalten jeweils einen Shunt $\underline{Z}_{s1}$ bzw. $\underline{Z}_{s2}$. Über die Messung der Spannung an den Shunts lassen sich die komplexen Stromstärken $I_{s1}$ und $I_{s2}$ für die beiden Kreisfrequenzen $\omega_1$ und $\omega_2$ bestimmen. Die Shunts sollten resistiv gewählt werden: da die Injektionssignale oberhalb der EKG-Bandbreite liegen, sind die vorwiegend kapazitiven Koppelimpedanzen $\underline{Z}_{drli}$, $\omega_i$ gegenüber dem Injektionssignal kleiner, als gegenüber dem EKG-Signal. Die resistiven Impedanzen der Shunts bleiben aber unverändert. Somit erzeugen diese im Frequenzband des EKG-Signales nur einen geringen Spannungsabfall und setzen somit die Effektivität der DRL-Elektrode nicht herab, während der Spannungsabfall im Band der Injektionssignale größer wird und somit eine präzisere Bestimmung von $\underline{I}_s$ erlaubt. Darüber hinaus besitzt das Modell die Eingangsimpedanzen $\underline{Z}_{in1}$ und $\underline{Z}_{in2}$, die die entsprechenden parasitären Eigenschaften der für die Shunt-Spannungsmessung verwendeten Verstärker modellieren.

Es soll nun gezeigt werden, dass mit den an den Shunts gemessenen Spannungen $\underline{U}_{s1,\omega_2}$ und $\underline{U}_{s2,\omega_1}$ sowie

$\underline{U}_{s1,\,\omega2}$ und $\underline{U}_{s2},\,\omega_2$ die am Patienten anliegende Spannung $\underline{U}_p,\,\omega_2$ sowie die beiden Koppelkapazitäten $\underline{Z}_{drl1},\,\omega_2$ $\underline{Z}_{drl2},$ $\omega_2$ der DRL-Elektrode bestimmt werden können. Das Verfahren, mit dessen Hilfe die zu den jeweiligen Injektionssignalen $\underline{U}_{inj1}$ und $\underline{U}_{inj2}$ gehörende Frequenzanteile $\omega_1$ und $\omega_2$ aus dem Messsignal demoduliert werden können, wurde zuvor schon beschrieben. Bei der Bestimmung der Spannungen und Ströme mit dem Index $\omega_1$ wird die Spannungsquelle $\underline{U}_{inj2}$ als Kurzschluss angenommen, mit dem Index $\omega_2$ - $\underline{U}_{inj1}$. Zur Vereinfachung können im Folgenden anstelle der Impedanzen die entsprechenden Admittanzen verwendet werden. Zunächst sollen die komplexen Stromstärkern über die Koppelimpedanzen bestimmt werden. Die erste kirchhoffsche Regel liefert:

$$\underline{I}_{drl_1,\,\omega_i} = \underline{Y}_{s_1,\,\omega_i}\,\underline{U}_{s_1,\,\omega_i} - \left(\underline{U}_{inj_i} - \underline{U}_{s_1,\,\omega_i}\right)\underline{Y}_{in_1,\,\omega_i} \qquad \text{für } i = 1,2 \qquad (3.16)$$

$$\underline{I}_{drl_2,\,\omega_1} = \underline{U}_{s_2,\,\omega_1}\left(\underline{Y}_{in_2,\,\omega_1} + \underline{Y}_{s_2,\,\omega_1}\right) \qquad (3.17)$$

$$\underline{I}_{drl_2,\,\omega_2} = \underline{Y}_{s_2,\,\omega_2}\,\underline{U}_{s_2,\,\omega_2} - \left(\underline{U}_{inj_2} - \underline{U}_{s_2,\,\omega_2}\right)\underline{Y}_{in_2,\,\omega_2} \qquad (3.18)$$

Um die weitere Rechnung zu vereinfachen, werden ab hier zwei Annahmen gemacht:

- die beiden Shunts sind im Vergleich zu den Koppelimpedanzen so niederohmig, dass für beide Elektroden und Frequenzen $\underline{U}_{inj} \gg \underline{U}_s$ gilt. $\underline{U}_s$ werden ab hier vernachlässigt.
- die Kreisfrequenzen $\omega_1$ und $\omega_2$ liegen so nah beieinander, dass für alle Admittanzen $\underline{Y}_{w1} \approx \underline{Y}_{\omega2}$ gilt. Es wird ab hier angenommen, dass die Admittanzen frequenzunabhängig sind.

Die zweite kirchhoffsche Regel liefert:

$$0 = \underline{U}_{drl_2,\,\omega_2} - \underline{U}_{drl_1,\,\omega_2} = \frac{\underline{I}_{drl_2,\,\omega_2}}{\underline{Y}_{drl_2}} - \frac{\underline{I}_{drl_1,\,\omega_2}}{\underline{Y}_{drl_1}} \qquad (3.19)$$

$$\Leftrightarrow \qquad \underline{Y}_{drl_2} = \underline{Y}_{drl_1}\frac{\underline{I}_{drl_2,\,\omega_2}}{\underline{I}_{drl_1,\,\omega_2}} \qquad (3.20)$$

$$0 = -\underline{U}_{inj_1} + \underline{U}_{drl_1,\,\omega_1} - \underline{U}_{drl_2,\,\omega_1} = -\underline{U}_{inj_1} + \frac{\underline{I}_{drl_1,\,\omega_1}}{\underline{Y}_{drl_1}} - \frac{\underline{I}_{drl_2,\,\omega_1}}{\underline{Y}_{drl_2}} \qquad (3.21)$$

Das Einsetzen von 3.20 in 3.21 und Umstellung liefert:

$$\underline{Y}_{drl_1} = \frac{1}{\underline{U}_{inj_1}}\left(\underline{I}_{drl_1,\,\omega_1} - \frac{\underline{I}_{drl_2,\,\omega_1}\,\underline{I}_{drl_1,\,\omega_2}}{\underline{I}_{drl_2,\,\omega_2}}\right) \qquad (3.22)$$

Für die Spannung $\underline{U}_p$ gilt:

$$\underline{U}_{p,\,\omega_2} = \underline{U}_{inj_2} - \frac{\underline{I}_{drl_1,\,\omega_2}}{\underline{Y}_{drl_1}} = \frac{\underline{I}_{drl_2,\,\omega_2}}{\underline{Y}_{drl_2}} \qquad (3.23)$$

Damit können aus 3.22 und 3.20 die beiden Koppelimpedanzen der DRL-Elektrode und aus 3.23 der an der Biosignalquelle anliegende Anteil des Injektionssignals bestimmt werden.

**Patentansprüche**

1. System zur kapazitiven Erfassung von elektrischen Biosignalen von einer Biosignalquelle (2), wobei das System wenigstens eine kapazitive Messelektrode (30) und wenigstens eine mit der Messelektrode (30) gekoppelte elektronische Auswerteeinheit zur Auswertung der elektrischen Signale der Messelektrode (30) aufweist, und wobei das System ferner Mittel zur Überwachung der Güte der kapazitiven Kopplung der Messelektrode mit der Biosignalquelle aufweist, wobei die Mittel zur Überwachung der Güte der kapazitiven Kopplung zusätzlich zu der Messelektrode (30) oder den Messelektroden (30) wenigstens zwei elektrisch voneinander separierte Injektionselektroden (40, 41) zur Einspeisung von Injektionssignalen in die wenigstens eine Messelektrode (30) über die Biosignalquelle (2) aufweisen, wobei das System Signalgeneratoren (21, 22) zur Erzeugung eines ersten Injektionssignals und eines vom ersten Injektionssignal unterschiedlichen zweiten Injektionssignals aufweist, die mittels der Injektionselektroden (40, 41) über die Biosignalquelle (2) in die Messelektrode (30) eingespeist werden, und wobei das System eine Bestimmungseinheit (1) aufweist, die dazu eingerichtet ist, anhand der über die Messelektrode (30) empfangenen Signale anhand der darin enthaltenen, von den ersten und zweiten Injektionssignalen herrührenden Signalanteilen die Güte der kapazitiven Kopplung der Messelektrode (30) mit der Biosignalquelle (2) zu bestimmen.

2. System nach dem vorhergehenden Anspruch, **dadurch gekennzeichnet, dass** eine, mehrere oder alle Messelektroden (30) des Systems als textile kapazitive Elektroden ausgebildet sind, die in einer Textilstruktur eingebettet sind.

3. System nach einem der vorhergehenden Ansprüche, **dadurch gekennzeichnet, dass** eine, mehrere oder alle Messelektroden (30) des Systems als flexible kapazitive Elektroden mit leitfähigen Strukturen ausgebildet sind, wobei die leitfähigen Strukturen als gedruckte Strukturen ausgebildet sind.

4. System nach einem der vorhergehenden Ansprüche, **dadurch gekennzeichnet, dass** die Bestimmungseinheit (1) zur Bestimmung der Güte der kapazitiven Kopplung anhand der Amplitudenwerte und der Phasenlagen der über die Messelektrode (30) empfangenen Signalanteile des ersten und des zweiten Injektionssignals eingerichtet ist.

5. System nach einem der vorhergehenden Ansprüche, **dadurch gekennzeichnet, dass** das System zur Bestimmung der Herzfrequenz oder einer daraus abgeleiteten Größe der Biosignalquelle (2) eingerichtet ist.

6. System nach einem der vorhergehenden Ansprüche, **dadurch gekennzeichnet, dass** der Bestimmungseinheit (1) als weitere Eingangsgrößen Messwerte der über die erste und die zweite Injektionselektrode (40, 41) mittels der zugeführten Injektionssignale eingespeisten Ströme zugeführt sind und die Bestimmungseinheit (1) zur Bestimmung der Güte der kapazitiven Kopplung unter Berücksichtigung der zugeführten Messwerte der in die erste und die zweite Injektionselektrode (40, 41) mittels der zugeführten Injektionssignale eingespeisten Ströme eingerichtet ist.

7. Verfahren zur Bestimmung der Güte der Kopplung wenigstens einer Messelektrode eines Systems nach einem der vorhergehenden Ansprüche mit einer Biosignalquelle, mit den Schritten:

   a) Zeitlich überlappendes oder gleichzeitiges Einspeisen eines ersten Injektionssignals und eines davon verschiedenen zweiten Injektionssignals über die Biosignalquelle in die wenigstens eine Messelektrode.
   b) Auswerten der von der wenigstens eine Messelektrode empfangenen elektrischen Signale,
   c) Bestimmen der Güte der kapazitiven Kopplung anhand der in den empfangenen Signalen enthaltenen Signalanteile des ersten und des zweiten Injektionssignals.

8. Computerprogramm mit Programmcodemitteln, eingerichtet zur Ausführung des Verfahrens nach dem Anspruch 7, wenn das Computerprogramm auf einem Rechner des Systems nach den Ansprüchen 1-6 ausgeführt wird.

**Claims**

1. System for capacitive detection of electrical biosignals from a biosignal source (2), wherein the system comprises at least one capacitive measuring electrode (30) and at least one electronic evaluation unit coupled to the measuring electrode (30) for evaluating the electrical signals of the measuring electrode (30), and wherein the system further comprises means for monitoring the quality of the capacitive coupling of the measuring electrode with the biosignal source, wherein the means for monitoring the quality of the capacitive coupling comprise, in addition to the measuring electrode (30) or the measuring electrodes (30), at least two injection electrodes (40, 41) for feeding injection signals into the at least one measuring electrode (30) via the biosignal source (2), the system having signal generators (21, 22) for generating a first injection signal and a second injection signal which is different from the first injection signal and is fed into the measuring electrode (30) by means of the injection electrodes (40, 41) via the biosignal source (2) and the system having a determination unit (1) which is set up to determine the quality of the capacitive coupling of the measuring electrode (30) to the biosignal source (2) on the basis of the signals received via the measuring electrode (30) and on the basis of the signal components contained therein which originate from the first and second injection signals.

2. System according to the preceding claim, **characterized in that** one, several or all measuring electrodes (30) of the system are formed as textile capacitive electrodes embedded in a textile structure.

3. System according to any one of the preceding claims, **characterized in that** one, several or all of the measuring electrodes (30) of the system are formed as flexible capacitive electrodes with conductive structures, wherein the conductive structures are formed as printed structures.

4. System according to any one of the preceding claims, **characterized in that** the determination unit (1) is set up to determine the quality of the capacitive coupling on the basis of the amplitude values and the phase positions of the signal components of the first and second injection signals received via the measuring electrode (30).

5. System according to any one of the preceding claims, **characterized in that** the system is arranged to determine the heart rate or a quantity derived therefrom of the biosignal source (2).

6. System according to one of the preceding claims, **characterized in that** measured values of the currents fed via the first and the second injection electrodes (40, 41) by means of the fed injection signals are fed to the determination unit (1) as further input variables, and the determination unit (1) is set up to determine the quality of the capacitive coupling taking into account the fed measured values of the currents fed into the first and the second injection electrodes (40, 41) by means of the fed injection signals.

7. Method of determining the quality of coupling of at least one measuring electrode of a system according to any one of the preceding claims with a biosignal source, comprising the steps of:

   a) Temporally overlapping or simultaneous injection of a first injection signal and a second injection signal different therefrom via the biosignal source into the at least one measuring electrode.
   b) Evaluating the electrical signals received from the at least one measuring electrode,
   c) Determining the quality of the capacitive coupling based on the signal components of the first and second injection signals contained in the received signals.

8. Computer program having program code means arranged to perform the method of claim 7 when the computer program is executed on a computer of the system of claims 1-6.


**Revendications**

1. Système de détection capacitive de biosignaux électriques provenant d'une source de biosignaux (2), le système comportant au moins une électrode de mesure capacitive (30) et au moins une unité d'évaluation électronique couplée à l'électrode de mesure (30) pour évaluer les signaux électriques de l'électrode de mesure (30), et le système comportant en outre des moyens de surveillance de la qualité du couplage capacitif de l'électrode de mesure à la source de biosignaux, les moyens de surveillance de la qualité du couplage capacitif comprennent, outre l'électrode de mesure (30) ou les électrodes de mesure (30), au moins deux électrodes d'injection (40, 41) séparées électriquement l'une de l'autre pour injecter des signaux d'injection dans ladite au moins une électrode de mesure (30) via ladite source de biosignaux (2), le système comportant des générateurs de signaux (21, 22) pour produire un premier signal d'injection et un deuxième signal d'injection différent du premier signal d'injection, qui sont injectés dans l'électrode de mesure (30) au moyen des électrodes d'injection (40, 41) via la source de biosignaux (2), et le système comportant une unité de détermination (1) qui est conçue pour déterminer la qualité du couplage capacitif de l'électrode de mesure (30) à la source de biosignaux (2), sur la base des signaux reçus par l'intermédiaire de l'électrode de mesure (30), sur la base des composantes de signal qui y sont contenues et qui proviennent des premier et deuxième signaux d'injection.

2. Système selon la revendication précédente,
   **caractérisé en ce qu'**une, plusieurs ou toutes les électrodes de mesure (30) du système sont réalisées sous forme d'électrodes capacitives textiles qui sont noyées dans une structure textile.

3. Système selon l'une des revendications précédentes,

**caractérisé en ce qu'**une, plusieurs ou toutes les électrodes de mesure (30) du système sont réalisées sous forme d'électrodes capacitives flexibles ayant des structures conductrices, les structures conductrices étant réalisées sous forme de structures imprimées.

4.  Système selon l'une des revendications précédentes,
    **caractérisé en ce que** l'unité de détermination (1) est conçue pour déterminer la qualité du couplage capacitif en se basant sur les valeurs d'amplitude et sur les positions de phase des composantes de signal des premier et deuxième signaux d'injection reçues par l'intermédiaire de l'électrode de mesure (30).

5.  Système selon l'une des revendications précédentes,
    **caractérisé en ce que** le système est conçu pour déterminer la fréquence cardiaque ou une variable, dérivée de celle-ci, de la source de biosignaux (2).

6.  Système selon l'une des revendications précédentes,
    **caractérisé en ce que** des valeurs mesurées des courants injectés par les première et deuxième électrodes d'injection (40, 41) au moyen des signaux d'injection alimentés sont amenées à l'unité de détermination (1) en tant que variables d'entrée supplémentaires, et l'unité de détermination (1) est conçue pour déterminer la qualité du couplage capacitif en tenant compte des valeurs mesurées alimentées des courants injectés dans les première et deuxième électrodes d'injection (40, 41) au moyen des signaux d'injection alimentés.

7.  Procédé de détermination de la qualité du couplage d'au moins une électrode de mesure d'un système selon l'une des revendications précédentes à une source de biosignaux, comprenant les étapes consistant à :

    a) injecter à chevauchement temporel ou simultanément un premier signal d'injection et un deuxième signal d'injection différent de celui-ci via la source de biosignaux dans ladite au moins une électrode de mesure,
    b) évaluer les signaux électriques reçus par ladite au moins une électrode de mesure,
    c) déterminer la qualité du couplage capacitif sur la base des composantes de signal des premier et deuxième signaux d'injection contenues dans les signaux reçus.

8.  Programme d'ordinateur comprenant des moyens de code de programme conçus pour mettre en œuvre le procédé selon la revendication 7, lorsque le programme d'ordinateur est exécuté sur un ordinateur du système selon les revendications 1 à 6.

Fig. 1

30

80

8   81   82

67

68

61

64

62

65

63

66

72

69

73

71

70

Fig. 2

Fig. 3

Fig. 4

Fig. 5

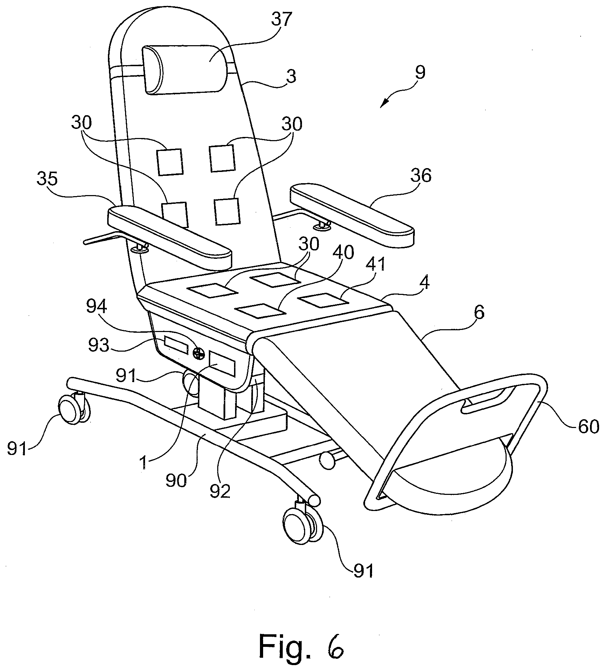

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013108810 A1
- EP 2101408 A1
- WO 2015075692 A1